# EUROPEAN PATENT SPECIFICATION

(11) **EP 4 136 250 B1**
(45) Date of publication and mention of the grant of the patent: **03.12.2025**
(21) Application number: 21788176.2
(22) Date of filing: 15.04.2021
(51) Int. Cl.: C12Q 1/04, C12M 1/34, G01N 15/14, G01N 21/64, G01N 33/58

(54) **ATTENUATED-BACKGROUND MICROBIOLOGICAL NUTRIENT MEDIA AND METHODS OF USING THE SAME**
MIKROBIOLOGISCHES NÄHRSTOFFMEDIUM MIT ABGESCHWÄCHTEM HINTERGRUND UND VERFAHREN ZUR VERWENDUNG DAVON
MILIEUX NUTRITIFS MICROBIOLOGIQUES À FOND ATTÉNUÉ ET PROCÉDÉS D'UTILISATION DE CES MILIEUX

(30) Priority: 15.04.2020 US 202063010337 P
(43) Date of publication of application: 22.02.2023
(73) Proprietor: Rapid Micro Biosystems, Inc., Lowell, MA 01854 (US)
(72) Inventor: BARRA, Jessica Tse, Lowell, MA 01854 (US); RODRIGUEZ SANTANA, Juan Pablo, Lowell, MA 01854 (US); GRABAR, Tammy Bohannon, Lowell, MA 01854 (US); RIPPETH, John, Melbourn, Herts SG8 6DP (GB); NOBLE, Michael, Melbourn, Herts SG8 6DP (GB); HUGHES, Gwilym, Melbourn, Herts SG8 6DP (GB)
(74) Representative: Simmons & Simmons LLP (Munich)
(86) International application number: PCT/US2021/027483
(87) International publication number: WO 2021/211845

(56) References cited:
- US-A- 3 814 670
- US-A- 5 403 359
- US-A- 5 622 857
- US-A- 5 811 312
- US-A1- 2002 191 824
- US-A1- 2015 072 377
- US-A1- 2015 368 690
- US-A1- 2017 218 426
- LONDON ROANNA ET AL: "An Automated System for Rapid Non-Destructive Enumeration of Growing Microbes", PLOS ONE, vol. 5, no. 1, 7 January 2010 (2010-01-07), pages e8609, XP093094316, DOI: 10.1371/journal.pone.0008609
- JON FLITNEY: "OXOID QUALITY ASSURANCE PRODUCT SPECIFICATION-CAMPYLOBACTER BLOOD-FREE SELECTIVE AGAR BASE (Modified CCDA-Preston)", THERMOFISCHER-PRODUCT SPECIFICATION FOR CM0739, 9 June 2012 (2012-06-09), XP055866899, Retrieved from the Internet <URL:http://tools.thermofisher.com/content/sfs/brochures/BT-SPEC-0200.pdf> [retrieved on 20211129]

## Description

### Background

In many industries, particularly the food, beverage, healthcare, electronic, and pharmaceutical industries, it is essential to analyze samples for the degree of contamination by microorganisms, such as bacteria, yeasts, or molds, rapidly. In particular, pharmaceutical and biologics companies are required to test cleanrooms and sterile products for the presence of microbiological contaminants by attempting to culture any microorganisms using nutrient media. London et al. in "An Automated System for Rapid NonDestructive Enumeration of Growing Microbes", 2010, PLOS ONE 5(1): e8609 disclose a method for detecting and enumerating growing microcolonies before they become visible to the eye using digital imaging of cellular autofluorescence. However, most nutrient media have background that interferes with optical detection methods. Accordingly, there is a need for a nutrient medium with reduced background.

### Summary of the Invention

The invention is defined in the claims. Subject-matter not encompassed by the claims is disclosed for reference. The invention features a microbiological nutrient medium that contains a dye and/or pigment that reduces background, e.g., allowing more rapid and/or higher sensitivity detection of microorganisms in a sample. The invention features a liquid, solid, or semi-solid nutrient medium containing at least one dye and/or pigment. A solid or semi-solid medium also includes a gelling agent. The nutrient medium supports the growth of a microorganism, and the at least one dye and/or pigment substantially reduces background of the medium. The medium comprises the pigment, wherein the pigment comprises carbon particles or iron oxide particles, wherein the carbon particles have an average diameter of less than 500 nm.

In one aspect, the invention features a liquid, solid, or semi-solid nutrient medium including at least one dye and/or pigment, wherein the nutrient medium supports the growth of a microorganism, wherein the at least one dye and/or pigment substantially reduces background of the medium, and wherein the solid or semi-solid nutrient medium further includes a gelling agent.

In one embodiment, the nutrient medium is disposed in a container, e.g., a dish, plate, or slide. In another embodiment, the nutrient medium is proud relative to the container. In another embodiment, the nutrient medium is rounded. In one embodiment, the nutrient medium includes a flat portion. In another embodiment, the nutrient medium further includes a circumferential portion that is sloped.

In one embodiment, the at least one dye and/or pigment renders the medium black. In another embodiment, wherein the at least one dye and/or pigment substantially reduces red background, orange background, yellow background, green background, blue background, or violet background in the nutrient medium from exposure to white light. In another embodiment, the at least one dye and/or pigment substantially reduces background in the nutrient medium from exposure to blue light. In one embodiment, the at least one dye and/or pigment provides a peak optical density in the nutrient medium of at least 0.004, e.g., at least 0.022, 0.046, 0.071, 0.097, 0.125, 0.155, 0.187, 0.222, 0.260, 0.301, 0.347, 0.398, 0.456, 0.523, 0.602, 0.699, 0.824, 1, 1.30, 2, 3, 4, 5, or 6.

In the invention, the nutrient medium includes the pigment. In the invention, the pigment includes carbon particles or iron oxide particles. In the invention, the carbon particles have an average diameter of less than 500 nm. In another embodiment, the carbon particles have an average diameter of between about 100 nm and about 400 nm. In one embodiment, the invention provides carbon particles including carbon black.

In another embodiment, the nutrient medium also includes a dye. In another embodiment, the dye comprises an azo dye. In another embodiment, the azo dye is acid black 71, acid black 194, acid black 2, reactive black 5, direct black 36, or Nyanza black.

In one embodiment, the nutrient medium further includes a polymer that binds to the at least one dye and/or pigment. In another embodiment, the polymer includes polydiallyldimethylammonium chloride (polyDADMAC), starch, cellulose, chitosan, β-cyclodextrin, calix[4]arene-based polymers, or a p-*tert-*butylcalix[4]arene-based polymer.

In one embodiment, the nutrient medium further includes a free radical scavenger, e.g., selected from the group consisting of N-acetyl-L-cysteine, sodium ascorbate, thiourea, nicotinamide, pyridoxine, sodium pyruvate, D-mannitol, 2-oxo-4-thiomethylbutyric acid (OBTA), 3-(methylthio) propionaldehyde (methional), dimethyl sulfoxide (DMSO), tert-butyl alcohol, benzoate, catalase, NADH peroxidase, thiol peroxidase, bacterioferritin comigratory protein, alkyl hydroperoxide reductase, glutathione peroxidase, cytochrome c peroxidase, and rubrerythrin.

In one embodiment, the microorganism is a bacterium, fungus, protist, or archaea, e.g., belonging to a genus selected from the group consisting of *Acinetobacter, Aspergillus, Bacillus, Burkholderia, Campylobacter, Candida, Clostridium, Corynebacterium, Delftia, Dermacoccus, Escherichia, Exserohilum, Helicobacter, Kocuria, Legionella, Listeria, Methylobacterium, Micrococcus, Mycobacterium, Paenibacillus, Penicillium, Propionibacterium, Pseudomonas, Ralstonia, Salmonella, Selenomonas, Serratia, Shigella, Staphylococcus, Stenotrophomonas, Streptococcus, Streptomyces, Vibrio,* and *Yersinia.*

In one embodiment, the gelling agent is selected from the group consisting of agar, gellan, sodium alginate, xanthan gum, guar gum, gelatin, agarose, polyacrylamide, and a polysaccharide produced by *Rhizobium sp.* (CNCM number: I-1809).

In another embodiment, the nutrient medium includes nutrients selected from the group consisting of trypticase soy medium (TSM), fluid thioglycollate medium (FTM), Schaedler medium, lysogeny/Lennox (LB) broth, mannitol yeast extract peptone broth, terrific broth II, Super Optimal broth with catabolite repression (SOC) medium, Thornton's medium, Lowenstein-Jensen medium, Sabouraud dextrose medium, D/E neutralizing medium, Loeffler medium, nalidixic acid and colimycin (ANC) medium, and artificial sea water medium. In one embodiment, the nutrient medium includes nutrients from bismuth sulfite agar, Chapman agar, Mueller-Hinton agar (MHA), blood agar, MacConkey agar, trypticase soy agar (TSA), Sabouraud dextrose agar (SDA), Reasoner's 2A agar (R2A), Mueller-Miller tellurite agar, or Tinsdale tellurite agar.

In another embodiment, the nutrient medium is solid or semi-solid. In one embodiment, the nutrient medium is liquid. In another embodiment, the nutrient medium is disposed within a porous matrix.

In one embodiment, the nutrient medium has a pH value between about pH 3.0 and about pH 12.0.

In one aspect, the invention provides a method of detecting the growth of a microorganism by contacting a surface of the nutrient medium with a sample under appropriate conditions to allow microbial growth; and optically detecting the surface to determine any growth of the microorganism. In one embodiment, the contacting includes placing a membrane containing the sample in contact with the surface. In another embodiment, the contacting includes contacting another surface with the surface. In another embodiment, the contacting includes allowing air to contact the surface.

In one embodiment, the microorganism belongs to a genus selected from the group consisting of *Acinetobacter, Aspergillus, Bacillus, Burkholderia, Campylobacter, Candida, Clostridium, Corynebacterium, Delftia, Dermacoccus, Escherichia, Exserohilum, Helicobacter, Kocuria, Legionella, Listeria, Methylobacterium, Micrococcus, Mycobacterium, Paenibacillus, Penicillium, Propionibacterium, Pseudomonas, Ralstonia, Salmonella, Selenomonas, Serratia, Shigella, Staphylococcus, Stenotrophomonas, Streptococcus, Streptomyces, Vibrio,* and *Yersinia.*

In another embodiment, optically detecting comprises detecting fluorescence.

### Definitions

The term "about," as used herein, refers to a value that is ±10% of the recited value.

The term "background," as used herein, refers to light scattered, reflected, or emitted from growth media in response to incident light in the absence of microorganisms.

The term "dye," as used herein, refers to a colorant substance that is soluble in water.

The term "label," as used herein, refers to an exogenous substance that can be detected optically.

The term "optical density," as used herein, refers to the attenuation of light (log (I₀/I) per centimeter exhibited by a dye and/or pigment. "Peak optical density," as used herein, refers to the optical density at the absorption maximum.

The term "pigment," as used herein, refers to a colorant substance that is insoluble in water.

The term "substantially reduces," as used herein, refers to a reduction of background by at least 50% relative to nutrient media without a dye and/or pigment, as described herein. In particular, the reduction of background may be at least 60, 65, 70, 75, 80, 85, 90, 95, or 99%. Reduction may occur at one or more wavelengths, e.g., over a range of wavelengths.

### Brief Description of the Drawings

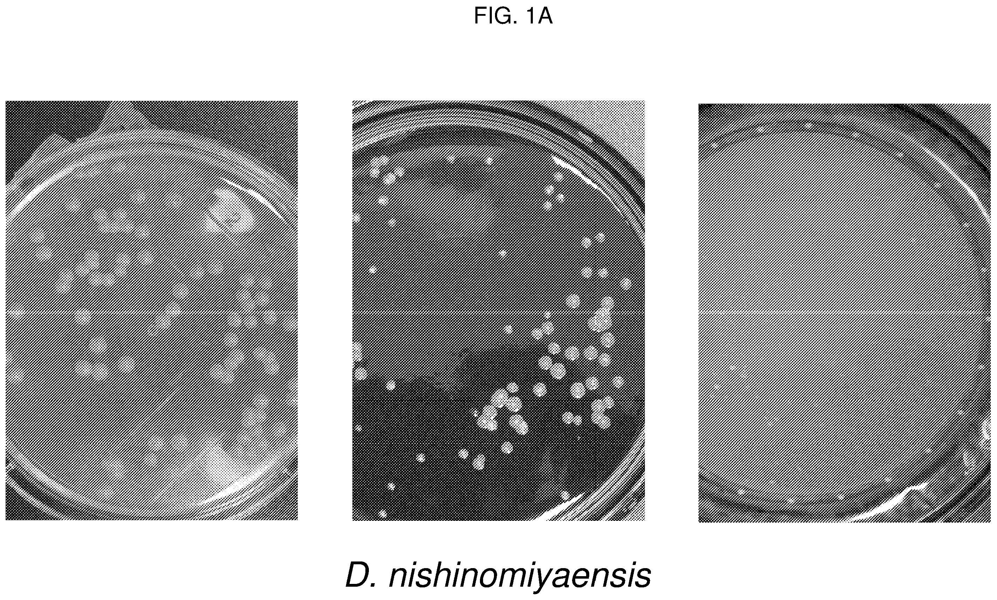

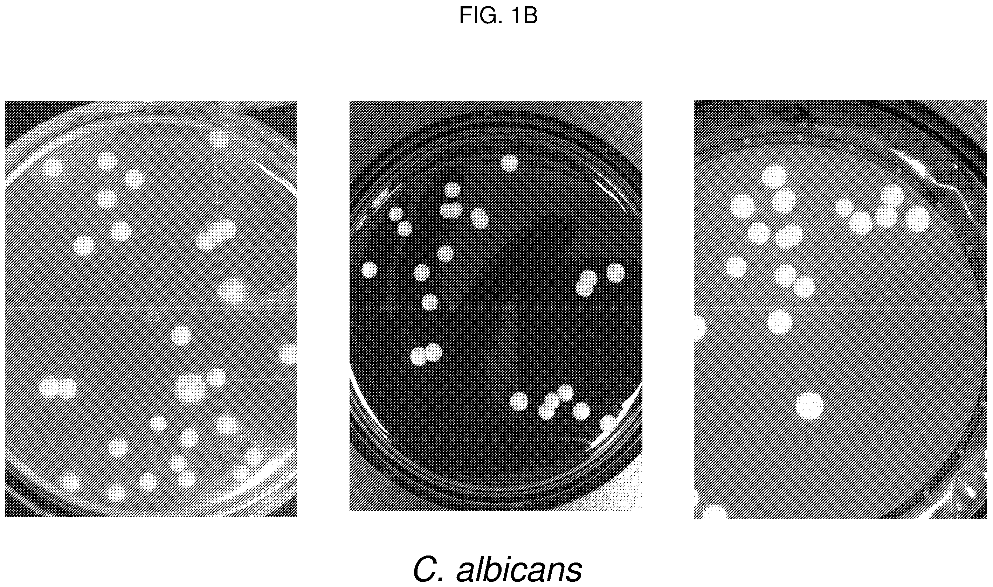

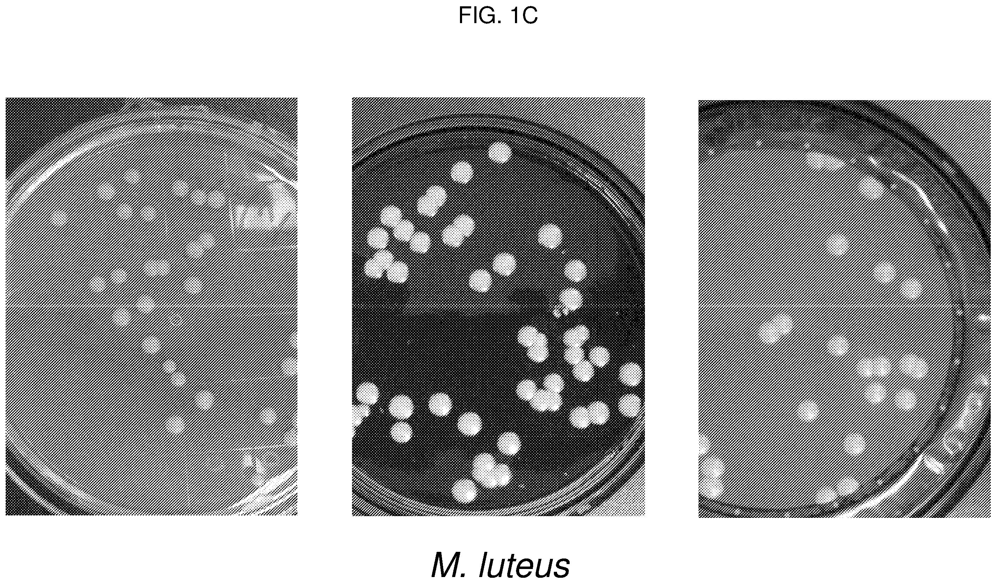

FIGS. 1A-1C are photographs of colonies grown on different growth media, RODAC plates (Becton Dickinson) *left,* medium of the invention *center,* and TSA medium covered by a black membrane (cassette from Rapid Micro Biosystems, Inc. Lowell, MA) *right.* FIG. 1A shows D. *nishinomiyaensis;* FIG. 1B shows *C*. *albicans;* and FIG. 1C shows *M*. *luteus.*

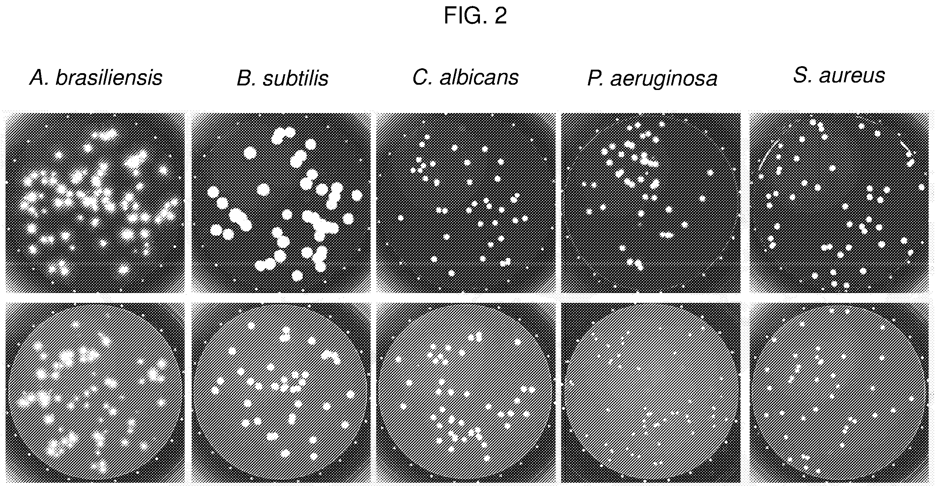

FIG. 2 is a set of photographs showing microbial colonies grown on a medium of the invention (top row) and colonies grown on TSA medium with membrane (bottom row). *A. brasiliensis, B. subtilis, C. albicans, P. aeruginosa,* and *S. aureus* were grown on the media.

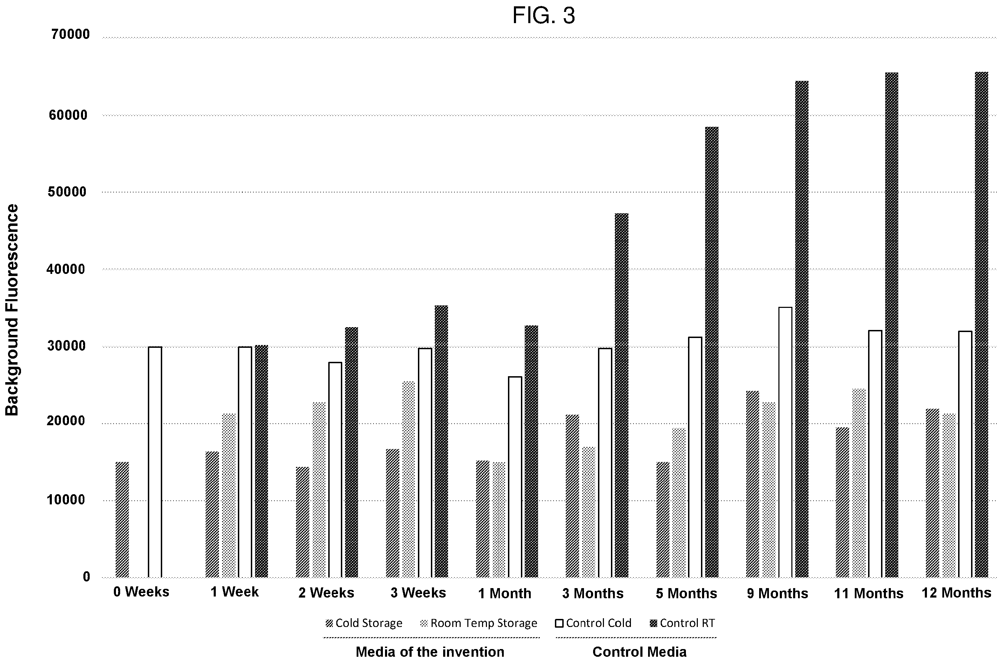

FIG. 3 is a graph showing background fluorescence levels of Nanoshel^{®} carbon black and TSA with lecithin and polysorbate 80 (TSA-LP80) cassettes (Black Media) and control TSA-LP80 cassettes with membrane (Control Media). Background fluorescence levels of the media were measured after storage in cold room or at room temperature for the indicated time periods.

### Detailed Description

This invention provides nutrient medium and methods of its use for optical detection of microorganisms as defined in the claims. The nutrient medium of the invention includes a dye and/or pigment that reduces background of the medium, which aids in optical detection, e.g., by eye or by automated detection. The medium is advantageous in increasing shelf life and stability under various storage temperatures and/or reducing the time to detection of certain microorganism and/or in detecting slow growing microorganisms and/or increasing recovery from different surfaces for microbiological monitoring.

The nutrient medium of the invention is useful in the detection of microorganisms, e.g., in the growth or sensitivity of microorganisms. The dye and/or pigment substantially reduces background of the medium, which increases the ability to detect microorganisms.

Microorganisms may be detected by any suitable optical method, e.g., by eye, fluorescence, phosphorescence, luminescence, absorbance, light scattering, or reflectance. Detection may be of an innate optical property of the microorganism, e.g., autofluorescence, bioluminescence, color, phosphorescence, light scattering, absorption, or reflectance, or from a label, e.g., a fluorescent label or a chemiluminescent label. In one embodiment, detection is of autofluorescence induced by blue light, e.g., as employed in the GrowthDirect^{®} automated detection system.

External illumination may or may not be employed in detection. For certain modalities, a specific wavelength or range of wavelengths of external illumination may be required, e.g., for detection of fluorescence or phosphorescence. In other modalities, non-specific wavelengths may be employed, e.g., for detection of reflectance. In other modalities, no external illumination is required, e.g., for detection of bioluminescence or chemiluminescence. One skilled in the art can select the appropriate light source, if used, for the detection method, e.g., LED lights, lasers, and gas discharge lamps. Broad spectrum lamps may also be employed with appropriate spectral filters. Signal from microorganisms may range across the UV to NIR range, typically within the visible range.

The nature of the dye and/or pigment employed may differ depending on the desired method of optical detection. For example, dyes or pigments that reduce background from a broad spectral region, e.g., across the visual range, may be employed. Alternatively, the dye and/or pigment may reduce background from a narrower spectral region for specific signals from microorganisms or combinations of signals and incident light. For example, the dye and/or pigment may absorb or scatter incident light to prevent excitation of a component of the nutrient medium. Alternatively, the dye and/or pigment may absorb or scatter light emitted by the nutrient medium in response to incident light. Combinations of such mechanisms are also envisioned by the invention.

A dye and/or pigment, or combination thereof, may appear black, e.g., to absorb or scatter substantially across the visible range. Alternatively, a dye and/or pigment, or combination thereof, may substantially reduce background in one more of the following spectral ranges: red background (625-740 nm), orange background (590-625 nm), yellow background (565-590 nm), green background (495-565 nm), blue background (400-495 nm), or violet background (380-400 nm).

A dye and/or pigment will be present in an amount sufficient to substantially reduce the desired background. Typically, the optical density (OD) of the dye and/or pigment in the nutrient medium is between 0.004 and 6, e.g., at least 0.022, 0.046, 0.071, 0.097, 0.125, 0.155, 0.187, 0.222, 0.260, 0.301, 0.347, 0.398, 0.456, 0.523, 0.602, 0.699, 0.824, 1, 1.30, 2, 3, 4, or 5, or at most 6, 5, 4, 3, 2, 1.3 or 1, such as between 1 and 6.

### Dyes, Pigments, and Additional Components

Any suitable dye, pigment, or combination thereof as defined in the claims may be employed in the nutrient medium. The dye and/or pigment will reduce the background as well as not substantially interfere with growth of the microorganism to be detected. Preferably, the dye and/or pigment also does not transfer to a surface being tested. The dye and/or pigment may be of any suitable color, e.g., black, red, orange, yellow, green, blue, or violet. For example, the dye and/or pigment may render the medium visibly black. Pigments of the invention include nanoparticles, such as carbon nanoparticles, e.g., from carbon black, and iron oxide. The size of nanoparticles is less than 500 nm, e.g., 100-400 nm, e.g., about 1 nm, about 5 nm, about 10 nm, about 15 nm, about 20 nm, about 25 nm, about 50 nm, about 75 nm, about 80 nm, about 85 nm, about 90 nm, about 95 nm, about 100 nm, about 105 nm, about 110 nm, about 120 nm, about 130 nm, about 140 nm, about 150 nm, about 160 nm, about 170 nm, about 180 nm, about 190 nm, about 195 nm, about 200 nm, about 205 nm, about 210 nm, about 220 nm, about 230 nm, about 240 nm, about 250 nm, about 260 nm, about 270 nm, about 280 nm, about 290 nm, about 295 nm, about 300 nm, about 305 nm, about 310 nm, about 320 nm, about 330 nm, about 340 nm, about 350 nm, about 360 nm, about 370 nm, about 380 nm, about 390 nm, about 395 nm, about 400 nm, about 405 nm, about 410 nm, about 425 nm, about 450 nm, about 495 nm, or about 499 nm. In another embodiment, the particles have an average diameter of between about 100 nm and about 400 nm, e.g., between about 100 nm and about 300 nm, between about 100 nm and about 250 nm, between about 100 nm and about 200 nm, between about 100 nm and about 150 nm, between about 100 nm and about 125 nm, between about 150 nm and about 400 nm, between about 150 nm and about 350 nm, between about 150 nm and about 300 nm, between about 150 nm and about 250 nm, between about 150 nm and about 200 nm, between about 150 nm and about 175 nm, between about 200 nm and about 400 nm, between about 200 nm and about 375 nm, between about 200 nm and about 350 nm, between about 200 nm and about 325 nm, between about 200 nm and about 300 nm, between about 200 nm and about 250 nm, between about 300 nm and about 400 nm, between about 300 nm and about 350 nm, between about 350 nm and about 400 nm, or between about 375 nm and about 400 nm. In another embodiment, the carbon particles include carbon black, graphitized carbon black, graphitic carbon, turbostratic carbon, black carbon, soot, biochar, carbon nanotubes, graphene, carbon nanofibers or other light-absorbing carbonaceous particles. An exemplary concentration of nanoparticles is from about 0.25% (w/v) to about 2.5% (w/v), e.g., from about 0.25% (w/v) to about 0.85% (w/v), from about 0.25% (w/v) to about 1.5% (w/v), from about 0.30% (w/v) to about 0.75% (w/v), from about 0.30% (w/v) to about 2% (w/v), from about 0.35% (w/v) to about 0.65% (w/v), from about 0.35% (w/v) to about 1.75% (w/v), from about 0.40% (w/v) to about 0.75% (w/v), from about 0.40% (w/v) to about 2% (w/v), from about 0.45% (w/v) to about 0.85% (w/v), from about 0.45% (w/v) to about 1.5% (w/v), from about 0.50% (w/v) to about 0.75% (w/v), from about 0.50% (w/v) to about 1.5% (w/v), from about 0.50% (w/v) to about 2% (w/v), from about 0.55% (w/v) to about 0.90% (w/v), from about 0.55% (w/v) to about 1.75% (w/v), from about 0.60% (w/v) to about 0.95% (w/v), from about 0.60% (w/v) to about 2% (w/v), from about 0.65% (w/v) to about 1% (w/v), from about 0.65% (w/v) to about 1.75% (w/v), from about 0.70% (w/v) to about 1% (w/v), from about 0.70% (w/v) to about 1.7% (w/v), from about 0.75% (w/v) to about 1.5% (w/v), from about 0.75% (w/v) to about 2% (w/v), from about 0.80% (w/v) to about 1.2% (w/v), from about 0.80% (w/v) to about 1.8% (w/v), from about 0.85% (w/v) to about 1.5% (w/v), from about 0.85% (w/v) to about 2% (w/v), from about 0.90% (w/v) to about 1.5% (w/v), from about 0.90% (w/v) to about 1.75% (w/v), from about 0.95% (w/v) to about 1.5% (w/v), from about 0.95% (w/v) to about 2% (w/v), from about 1% (w/v) to about 1.8% (w/v), from about 1.5% (w/v) to about 2% (w/v), or from about 1.75% (w/v) to about 2% (w/v),.

Suitable dyes include azo dyes, e.g., acid black 71, acid black 194, reactive black 5, direct black 36, and Nyanza black. Another example is acid black 2. Combinations of various dyes and pigments are also envisioned. In certain embodiments, the dye and/or pigment does not change color due to a pH change. In other embodiments, the dye and/or pigment is not digestible by a microbial enzyme.

Certain dyes or pigments may be prone to transfer to surfaces when contacted by the nutrient medium of the invention. Accordingly, the nutrient medium may include a polymer that binds to the dye and/or pigment to reduce or eliminate transfer. An exemplary polymer is polydiallyldimethylammonium chloride (polyDADMAC). Other polymers include sugar based polymers (oligo-polysaccharides) such as starch, cellulose, chitosane, β-cyclodextrin, calix[4]arene-based polymers, and p-tert-butylcalix[4]arene-based polymers.

Nutrient media may also be sterilized by a variety of methods, e.g., autoclaving or gamma irradiation. Gamma irradiation may induce free radicals in the medium. Thus, nutrient media of the invention may also include a free radical scavenger. Examples of free radical scavengers include N-acetyl-L-cysteine, sodium ascorbate, thiourea, nicotinamide, pyridoxine, sodium pyruvate, D-mannitol, 2-oxo-4-thiomethylbutyric acid (OBTA), 3-(methylthio) propionaldehyde (methional), dimethyl sulfoxide (DMSO), tert-butyl alcohol, benzoate, and enzymes such as catalase, NADH peroxidase, thiol peroxidase, bacterioferritin comigratory protein, alkyl hydroperoxide reductase, glutathione peroxidase, cytochrome c peroxidase, and rubrerythrin.

### Gelling Agent and Matrices

The nutrient medium may include a gelling agent. Non-limiting examples of gelling agents include agar, gellan, sodium alginate, xanthan gum, guar gum, gelatin, agarose, polyacrylamide, Eladium^{™} (a polysaccharide produced by *Rhizobium sp.* (CNCM number: I-1809)), coagulated eggs, coagulated serum, and combinations thereof. The amount of the gelling agent in the solid composition of the invention depends on the identity of the gelling agent.

Alternatively or in addition, a nutrient medium may be absorbed or gelled inside a matrix, e.g., a porous matrix such as a foam pad, sponge, porous plastic, e.g., Porex, or paper.

### Growth Media Composition

Any suitable nutrients can be included in the nutrient medium of the invention. The nutrients selected will depend on the nature of the microorganisms being detected. Non-limiting examples of nutrients include those in trypticase soy medium (TSM), fluid thioglycollate medium (FTM), Schaedler medium, lysogeny/Lennox (LB) broth, mannitol yeast extract peptone broth, terrific broth II, Super Optimal broth with catabolite repression (SOC) medium, nalidixic acid and colimycin (ANC) medium, Lowenstein-Jensen medium, Sabouraud dextrose medium, D/E neutralizing medium, and Loeffler medium. Nutrients may also be the nutrients from any one of Thornton's medium, trypticase soy agar (TSA), Sabouraud dextrose agar (SDA), Reasoner's 2A agar (R2A), Mueller Hinton agar (MHA), bismuth sulfite agar, Chapman agar, blood agar, MacConkey agar, Mueller-Miller tellurite agar, and Tinsdale tellurite agar. Another suitable medium can be found in WO 2015/164827.

The nutrient medium of the invention can have any pH value, including an acidic, alkaline or neutral pH. An exemplary pH value of the nutrient medium is from about pH 3.0 to about pH 12.0, e.g., from about pH 3.0 to about pH 11.0, from about pH 3.5 to about pH 10.0, from about pH 4.0 to about pH 9.5, from about pH 4.5 to about pH 9.0, from about pH 5.0 to about pH 8.5, from about pH 5.5 to about pH 8.0, from about pH 5.5 to about pH 7.5, from about pH 5.5 to about pH 6.5, from about pH 6.0 to about pH 7.0, from about pH 6.5 to about pH 7.5, from about pH 6.8 to about pH 7.3, from about pH 7.0 to about pH 8.0, from about pH 7.0 to about pH 8.5, from about pH 7.0 to about pH 9.0, from about pH 7.0 to about pH 9.5, from about pH 7.0 to about pH 10.0, from about pH 7.0 to about pH 11.0, from about pH 7.0 to about pH 12.0, from about pH 8.0 to about pH 9.0, from about pH 8.0 to about pH 10.0, from about pH 8.0 to about pH 11.0, from about pH 8.0 to about pH 12.0, from about pH 9.0 to about pH 10.0, from about pH 9.0 to about pH 11.0, from about pH 9.0 to about pH 12.0, from about pH 10.0 to about pH 11.0, from about pH 11.0 to about pH 12.0, from about pH 3.0 to about pH 4.0, from about pH 4.0 to about pH 5.0, or from about pH 5.0 to about pH 6.0.

Any additives can be included in the nutrient medium of the invention. Such additives may improve the physico-chemical properties of the media without significantly negatively affecting the growth properties of the nutrient medium. Non-limiting examples of additives include buffers (e.g., TRIS and/or citrate), chelating agents (e.g., EDTA), antioxidants, detergents, surfactants (e.g., polysorbate 20), emulsifiers, disinfectant neutralizing agents (e.g., lecithin, polysorbate 80, histidine, thiosulfate, bisulfite, glycine, divalent cations (e.g., Mg²⁺ or Ca²⁺), and/or thioglycollate), micelle forming agents, micelle inhibiting agents and/or polypropylene glycol, polyethylene glycol and/or carboxymethylcellulose. The culture medium may further include an antibiotic, e.g., for susceptibility or resistance testing or for selection of resistant cells.

### Form of the Nutrient Medium

The nutrient medium may be in any suitable form or shape. In particular, nutrient medium can be provided in dry form, where liquids, e.g., water, are added to form a gel or broth. Alternatively, the nutrient medium can be provided in gel form. In gel form, the nutrient medium is typically housed in or on a container, e.g., plate, dish, slide, or other flat surface, e.g., with a bottom and lid. The nutrient medium may be proud relative to the container, e.g., plate or dish, i.e., extending above the bottom (and any sidewalls present) to be able to contact surfaces, or recessed in the bottom (e.g., below the edge of a sidewall). An exemplary thickness of the solid or semi-slid media in or on a container is from about 100 µm to about 2 cm, e.g., from about 100 µm to about 1.5 cm, from about 100 µm to about 1 cm, from about 150 µm to about 1.5 cm, from about 200 µm to about 1 mm, from about 200 µm to about 9 mm, from about 200 µm to about 2 cm, from about 250 µm to about 1.5 mm, from about 250 µm to about 7.5 mm, from about 300 µm to about 1 mm, from about 300 µm to about 1.5 cm, from about 400 µm to about 2 mm, from about 400 µm to about 5 mm, from about 400 µm to about 2 cm, from about 500 µm to about 1 mm, from about 500 µm to about 7 mm, from about 500 µm to about 1.5 cm, from about 600 µm to about 9 mm, from about 600 µm to about 2 cm, from about 700 µm to about 1.5 cm, from about 750 µm to about 7.5 mm, from about 800 µm to about 2.5 mm, from about 800 µm to about 1.75 cm, from about 900 µm to about 9 mm, from about 900 µm to about 2 cm, from about 1 mm to about 5 mm, from about 1 mm to about 7 mm, from about 1 mm to about 1 cm, from about 2 mm to about 6 mm, from about 2 mm to about 1.5 cm, from about 3 mm to about 9 mm, from about 3 mm to about 1.75 cm, from about 4 mm to about 1 cm, from about 4 mm to about 1.5 cm, from about 4 mm to about 2 cm, from about 5 mm to about 1.2 cm, from about 5 mm to about 1.8 cm, from about 6 mm to about 1.5 cm, from about 6 mm to about 2 cm, from about 7 mm to about 1.2 cm, from about 7 mm to about 1.9 cm, from about 8 mm to about 1.4 cm, from about 9 mm to about 1.75 cm, from about 1 cm to about 1.4 cm, from about 1 cm to about 1.8 cm, from about 1.1 cm to about 1.6 cm, from about 1.2 cm to about 1.7 cm, from about 1.3 cm to about 1.9 cm, from about 1.4 cm to about 2 cm, from about 1.5 cm to about 2 cm, or from about 1.7 cm to about 2 cm. The medium may be flat or rounded or include a flat portion, e.g., of 1 -15 cm in diameter. When the nutrient medium includes a flat portion, the edges of the flat portion may be vertical, i.e., at 90 degrees, or the medium may include a circumferential portion around the flat portion, where the circumferential portion is sloped, i.e., at less than 90 degrees, e.g., at most 85, 80, 75, 70, 65, 60, 55, 50, 45. 40, 35, 30, 25, 20, 15, or 10 degrees. The surface of the medium may also be concave or convex. Concave surfaces may provide ease of contacting other surfaces. The medium, e.g., when including a dye, may also be in the form of a liquid that may loaded into a matrix or employed in a dish or culture tube.

The dye and/or pigment may be mixed uniformly throughout the nutrient medium or may be present in a surface layer. For example, a layer of nutrient medium containing dye and/or pigment, e.g., between 0.1 and 10 mm, may be applied to a base layer of nutrient medium not containing dye and/or pigment.

### Microorganisms

Microorganisms include bacteria, fungi, protists, and archaea. Non-limiting examples of the genera of microorganisms (e.g., bacteria or fungi) that can be cultured using the nutrient media of the invention include *Acinetobacter* (e.g., *Acinetobacter lwofii*)*, Aspergillus* (e.g., *Aspergillus brasiliensis* or *Aspergillus fumigates), Bacillus* (e.g., *Bacillus clausii, Bacillus idriensis, Bacillus licheniformis,* or *Bacillus subtilis), Burkholderia* (e.g., *Burkholderia pseudomallei), Campylobacter* (e.g., *Campylobacter jejuni*), *Candida (e.g., Candida albicans), Clostridium* (e.g., *Clostridium botulinum* or *Clostridium perfringens), Corynebacterium* (e.g., *Corynebacterium tuberculostearicum* or *Corynebacterium xerosis), Delftia* (e.g., *Delftia acidovorans), Dermacoccus* (e.g., *Dermacoccus nishinomiyaensis), Escherichia* (e.g., *Escherichia coli*), *Exserohilum* (e.g., *Exserohilum rostratum*), *Helicobacter* (e.g., *Helicobacter pylon), Kocuria* (e.g., *Kocuria rhizophila), Legionella* (e.g., *Legionella pneumophila), Listeria* (e.g., *Listeria ivanovii, Listeria grayi,* or *Listeria monocytogenes), Methylobacterium* (e.g., *Methylobacterium radiotolerans), Micrococcus* (e.g., *Micrococcus luteus), Mycobacterium* (e.g., *Mycobacterium tuberculosis* or *Mycobacterium leprae), Paenibacillus* (e.g., *Paenibacillus glucanolyticus), Penicillium* (e.g., *Penicillium chrysogenum* or *Penicillium notatum), Propionibacterium* (e.g., *Propionibacterium acnes), Pseudomonas* (e.g., *Pseudomonas aeruginosa), Ralstonia* (e.g., *Ralstonia pickettii*), *Salmonella* (e.g., *Salmonella enterica* serotype Typhi), *Selenomonas* (e.g., *Selenomonas noxia), Serratia* (e.g., *Serratia marcescens), Shigella* (e.g., *Shigella flexneri*), *Staphylococcus* (e.g., *Staphylococcus aureus, Staphylococcus epidermidis,* or *Staphylococcus hominis), Stenotrophomonas* (e.g., *Stenotrophomonas maltophilia), Streptococcus* (e.g., *Streptococcus pyogenes), Streptomyces* (e.g., *Streptomyces halstedii*), *Vibrio* (e.g., *Vibrio cholerae, Vibrio parahaemolyticus,* or *Vibrio vulnificus),* and *Yersinia* (e.g., *Yersinia enterocolitica).*

Nutrient media may also be used to grow viruses inside cells, e.g., lytic viruses, where plaques form in a lawn of host organisms.

### Uses

Exemplary fields of use for the nutrient media of the invention include general cell culture and testing liquid, air, soil, surfaces, industrial or clinical samples, pharmaceutical products (sterile or non-sterile), food products, beverage products, cosmetics, personal care products, or nutritional supplements for microbial bioburden. The nutrient medium may also be employed in other assays, such as clinical assays, e.g., for blood or other infections, and assays for antibiotic resistance. Samples that can be assayed using the nutrient media of the invention are not limited and include industrial samples (e.g., raw, in-process, or finished material in the manufacture of foods, beverages, or nutritional supplements; raw, in-process, or finished material in the manufacture of medical or in vitro diagnostic devices; chemical products; industrial surfaces; instrumentation; or machinery), pharmaceuticals and reagent used in preparing pharmaceuticals (e.g., raw, finished, or in-process material in the manufacture of pharmacological, cosmetic, personal care, blood, or other products for topical or internal use in humans or animals), biological samples, environmental samples (e.g., water samples (such as natural bodies of water (such as rivers, lakes, ponds, and oceans), waste water, and treated sources of water (such as municipal water supplies)), air samples (passive or active), soil samples, and surface samples). Surfaces that may be tested include equipment, materials, and facilities used in the manufacture, packaging, or storage of goods (e.g., pharmaceuticals); equipment, materials, and facilities used in research; clothing, bedding, and other fabrics (e.g., for medical providers or patients); and equipment, materials, and facilities used in treatment (e.g., hospitals, clinics, and doctor's offices).

Samples may be placed on the nutrient medium by any suitable method. Such methods include direct contact, e.g., with a surface or passive or active air sampling. Additionally, the methods may include contact by any suitable instrument, e.g., loop, needle, or swab. Samples may also be added in liquid form to the nutrient media. A membrane through which a sample has been filtered may also be placed on the nutrient medium of the invention, where the dye and/or pigment reduces background from around the edges of the membrane or detectable through the membrane.

The nutrient medium of the invention may be employed in detecting microorganisms, e.g., by colony counting. Microbial growth on the nutrient medium of the invention is easier to detect optically, e.g., by eye or automatically, because of the reduced background. Easier optical detection of microbial growth on the nutrient medium may reduce the time to results (TTR) of certain microorganisms (see, e.g., Table 1). The nutrient medium may in particular be useful for rapid microbiology detection, e.g., in detecting microcolonies. For example, the nutrient medium of the invention may be employed in the detection of colonies having diameters of at least 10, 20, 30, 40, 50, 60, 70, 80, 90, 100, 200, 250, 300, 350, 400, 450, 500, 550, 600, 650, 700, or 750 µm, e.g., up to 1 mm.

| Table **1.Organism** |
|---|
| *Aspergillus brasiliensis* ATCC^{®} 16404 |
| *Bacillus cereus* Rapid Micro Biosystems (RMB) Isolate |
| *Bacillus circulans* RMB Isolate |
| *Bacillus megaterium* RMB Isolate |
| *Bacillus pumilus* RMB Isolate |
| *Bacillus subtilis* ATCC^{®} 6633 |
| *Burkholderia cepacia* ATCC^{®} 25608 |
| *Candida albicans* ATCC^{®} 10231 |
| *Escherichia coli* RMB Isolate |
| *Micrococcus luteus* Novartis Strain |
| *Penecillium chrysogenum* ATCC^{®} 10106 |
| *Pseudomonas fluorescens* RMB Isolate |
| *Pseudomonas aeruginosa* ATCC^{®} 9027 |
| *Ralstonia pickettii* Apptec or RMB Isolate |
| *Serratia marcescens* RMB Isolate |
| *Staphylococcus aureus* ATCC^{®} 6538 |
| *Staphylococcus epidermidis* ATCC^{®} 12228 |
| *Stenotrophomonas maltophilia* RMB Isolate |
| *Streptococcus pyogenes* ATCC^{®} 19615 |
| *Dermacoccus nishinomiyaensis* RMB Isolate |

### Examples

The following examples are meant to illustrate the invention and are not meant to limit the invention in any way.

### Example 1: Comparison of microorganism growth on nutrient medium of the invention to growth on media without dye and/or pigment

FIGS. 1A-1C show representative colonies for different microbial species cultured as per USP <1227> on cassettes from Rapid Micro Biosystems with a black filter over TSA with lecithin and polysorbate 80 (TSA-LP80) without pigment or dye added (*right*); on nutrient media of an embodiment of the invention, TSA-LP80 with 1% mesoporous carbon black nanopowder pigment, graphitized, <500nm particle size (Sigma Aldrich #699624) *(center);* and on BD RODAC^{™} nutrient media without dye and/or pigment added *(left).*

FIG. 1A shows the growth of *D*. *nishinomiyaensis.* FIG. 1B shows the growth of *C. albicans.* FIG. 1C shows the growth of *M. luteus.*

Representative images of microbial colonies grown on medium of the invention (top row) and colonies grown on cassettes from Rapid Micro Biosystems with TSA medium with membrane (bottom row) are shown in FIG. 2. *A. brasiliensis, B. subtilis, C. albicans, P. aeruginosa,* and *S. aureus* were grown on both media.

### Example 2: Comparison of recovery of microorganism growth on nutrient medium of the invention to growth on RODAC^{™} nutrient medium

Table 2 is a list of microorganisms cultured with their respective number of colony forming units (CFUs), as well as a percent recovery of CFUs from an embodiment of the invention compared to the BD RODAC^{™} nutrient media. For all organisms cultured, greater than 70% recovery was achieved with the nutrient media of the invention relative to BD RODAC^{™} nutrient media. Each test was performed in six replicates.

**Table 2.**

| Organism Cultured | Nutrient Medium | | | Carbon Black TSA-LP80 vs. RODAC (% Recovery) |
|---|---|---|---|---|
| | Carbon Black TSA-LP80 | Carbon Black TSA-LP80 | Carbon Black BD RODAC | |
| | Number of CFUs/Sample | | | |
| *Bacillus subtilis* | | | | |
| ATCC 6633 | | | | |
| 32.5°C, 24-48 hr | 48 | 53 | 49 | 98% |
| *Pseudomonas aeruginosa* | | | | |
| ATCC 9027 | | | | |
| 32.5°C, 24-48 hr | 39 | 47 | 40 | 98% |
| *Staphylococcus aureus* | | | | |
| ATCC 6538 | | | | |
| 32.5°C, 24-48 hr | 36 | 26 | 37 | 97% |
| *Micrococcus luteus* | | | | |
| 32.5°C, 48-72 hr | 29 | 29 | 27 | 107% |
| *Aspergillus brasiliensis* | | | | |
| ATCC 16404 | | | | |
| 32.5°C, 48-72 hr | 36 | 30 | 28 | 129% |
| *Candida albicans* | | | | |
| ATCC 10231 | | | | |
| 32.5°C, 48-72 hr | 29 | 30 | 27 | 107% |
| *Dermacoccus nishinomiyaensis* | | | | |
| 32.5°C, 72-120 hr | 74 | 62 | 67 | 110% |

### Example 3. Comparison of recovery of microorganism growth using nutrient media in contact testing

Table 3 provides the recovery of *B*. *subtilis* from sampling on different surface types (Surface). The number of CFUs obtained from each surface type is indicated for RODAC^{™} nutrient medium (RODAC), cassette with TSA-LP80, and black medium cassettes with 1% w/v Nanoshel^{®} carbon mesoporous nanopowder, <100 nm particle size (invention). The percent recovery of CFUs of the RMB standard cassette versus RODAC^{™} nutrient medium and the percent recovery of the black medium cassette versus RODAC^{™} nutrient medium are shown. The black medium cassette demonstrated >85% recovery for all surfaces tested compared to the RODAC^{™} nutrient medium.

**Table 3.**

| Surface | RODAC (CFU) | TSA-LP80 (CFU) | TSA-LP80 vs. RODAC (% Recovery) | Invention (CFU) | Invention vs. RODAC (% Recovery) |
|---|---|---|---|---|---|
| Glass Coupon | 90 | 103 | 114% | 97 | 107% |
| Lab Floor Coupon (Rough Surface) | 79 | 17 | 22% | 73 | 92% |
| Lab Coat | 77 | 61 | 80% | 68 | 89% |
| Air (Active Air) | 44 | 37 | 83% | 44 | 100% |

### Example 4. Comparison of fluorescence background levels between black media cassettes and control cassettes after storage at different temperatures

Background fluorescence levels of black media cassettes (Nanoshel^{®} carbon black and TSA with lecithin and polysorbate 80 (TSA-LP80)) and control TSA-LP80 cassettes were measured after cold room (4°C) or room temperature storage for the indicated time periods (i.e., between 0 weeks to 11 months) (FIG. 3). Control media stored at room temperature showed higher background fluorescence at later time points (e.g., at 3, 5, 9 and 11 months) compared to 1 week of storage; media of the invention showed no significant increase in background fluorescence even after storage at room temperature for 11 months.

## Claims

1. A liquid or solid or semi-solid nutrient medium comprising at least one pigment and optionally at least one dye, wherein the nutrient medium supports the growth of a microorganism, wherein the at least one pigment and optionally at least one dye, substantially reduces background of the medium, and wherein the solid or semi-solid nutrient medium further comprises a gelling agent, wherein the medium comprises the pigment, wherein the pigment comprises carbon particles or iron oxide particles, wherein the carbon particles have an average diameter of less than 500 nm.

2. The nutrient medium of claim 1, wherein the nutrient medium is disposed in a container, wherein optionally the nutrient medium is proud relative to the container, wherein optionally the nutrient medium is rounded.

3. The nutrient medium of claim 1, wherein the at least one pigment and optionally at least one dye, substantially reduces red background, orange background, yellow background, green background, blue background, or violet background from exposure to white light, or background from exposure to blue light.

4. The nutrient medium of claim 1, wherein the at least one pigment and optionally at least one dye, provides a peak optical density of between 0.004 and 6.

5. The nutrient medium of claim 1, wherein the carbon particles have an average diameter of between about 100 nm and about 400 nm.

6. The nutrient medium of claim 1, wherein the medium further comprises the dye, wherein optionally the dye comprises an azo dye, wherein optionally the azo dye is acid black 71, acid black 194, acid black 2, reactive black 5, direct black 36, or Nyanza black.

7. The nutrient medium of claim 1, further comprising a polymer that binds to the at least one pigment and optionally at least one dye, wherein optionally the polymer comprises polydiallyldimethylammonium chloride (polyDADMAC), starch, cellulose, chitosan, β-cyclodextrin, calix[4]arene-based polymers, or a p-tert-butylcalix[4]arene-based polymer.

8. The nutrient medium of claim 1, further comprising a free radical scavenger, wherein optionally the free radical scavenger is selected from the group consisting of N-acetyl-L-cysteine, sodium ascorbate, thiourea, nicotinamide, pyridoxine, sodium pyruvate, D-mannitol, 2-oxo-4-thiomethylbutyric acid (OBTA), 3-(methylthio) propionaldehyde (methional), dimethyl sulfoxide (DMSO), *tert*-butyl alcohol, benzoate, catalase, NADH peroxidase, thiol peroxidase, bacterioferritin comigratory protein, alkyl hydroperoxide reductase, glutathione peroxidase, cytochrome c peroxidase, and rubrerythrin.

9. The nutrient medium of claim 1, wherein the microorganism is a bacterium, fungus, protist, or archaea, wherein optionally the microorganism belongs to a genus selected from the group consisting of *Acinetobacter, Aspergillus, Bacillus, Burkholderia, Campylobacter, Candida, Clostridium, Corynebacterium, Delftia, Dermacoccus, Escherichia, Exserohilum, Helicobacter, Kocuria, Legionella, Listeria, Methylobacterium, Micrococcus, Mycobacterium, Paenibacillus, Penicillium, Propionibacterium, Pseudomonas, Ralstonia, Salmonella, Selenomonas, Serratia, Shigella, Staphylococcus, Stenotrophomonas, Streptococcus, Streptomyces, Vibrio,* and *Yersinia.*

10. The nutrient medium of claim 1, wherein the gelling agent is selected from the group consisting of agar, gellan, sodium alginate, xanthan gum, guar gum, gelatin, agarose, polyacrylamide, and a polysaccharide produced by *Rhizobium sp.* (CNCM number: I-1809).

11. The nutrient medium of claim 1, further comprising nutrients selected from the group consisting of trypticase soy medium (TSM), fluid thioglycollate medium (FTM), Schaedler medium, lysogeny/Lennox (LB) broth, mannitol yeast extract peptone broth, terrific broth II, Super Optimal broth with catabolite repression (SOC) medium, Thornton's medium, Lowenstein-Jensen medium, Sabouraud dextrose medium, D/E neutralizing medium, Loeffler medium, nalidixic acid and colimycin (ANC) medium, artificial sea water medium, bismuth sulfite agar, Chapman agar, Mueller-Hinton agar (MHA), blood agar, MacConkey agar, trypticase soy agar (TSA), Sabouraud dextrose agar (SDA), Reasoner's 2A agar (R2A), Mueller-Miller tellurite agar, or Tinsdale tellurite agar.

12. The nutrient medium of any one of claims 1-11, wherein the nutrient is solid or semi-solid.

13. The nutrient medium of any one of claims 1-11, wherein the nutrient medium is liquid, wherein optionally the nutrient medium is disposed within a porous matrix.

14. A method of detecting the growth of a microorganism comprising:
(a) contacting a surface of the nutrient medium of claim 12 with a sample; and
(b) optically detecting fluorescence on the surface to determine any growth of the microorganism.

15. The method of claim 14, wherein step (a) comprises placing a membrane containing the sample in contact with the surface, or wherein step (a) comprises contacting an external surface with the surface, and/or wherein step (a) comprises allowing air to contact the surface.

## Patentansprüche

1. Flüssiges, festes oder halbfestes Nährmedium, das mindestens ein Pigment und optional mindestens einen Farbstoff enthält, wobei das Nährmedium das Wachstum eines Mikroorganismus unterstützt, wobei das mindestens eine Pigment und optional mindestens ein Farbstoff den Hintergrund des Mediums erheblich reduzieren und wobei das feste oder halbfeste Nährmedium ferner ein Geliermittel umfasst, wobei das Medium das Pigment umfasst, wobei das Pigment Kohlenstoffpartikel oder Eisenoxidpartikel umfasst, wobei die Kohlenstoffpartikel einen durchschnittlichen Durchmesser von weniger als 500 nm aufweisen.

2. Nährmedium nach Anspruch 1, wobei das Nährmedium in einem Behälter angeordnet ist, wobei das Nährmedium optional gegenüber dem Behälter hervorsteht, wobei das Nährmedium optional abgerundet ist.

3. Nährmedium nach Anspruch 1, wobei das mindestens eine Pigment und optional mindestens ein Farbstoff den roten Hintergrund, den orangefarbenen Hintergrund, den gelben Hintergrund, den grünen Hintergrund, den blauen Hintergrund oder den violetten Hintergrund durch Belichtung mit weißem Licht oder den Hintergrund durch Belichtung mit blauem Licht wesentlich reduziert.

4. Nährmedium nach Anspruch 1, wobei das mindestens eine Pigment und optional mindestens ein Farbstoff eine maximale optische Dichte zwischen 0,004 und 6 aufweisen.

5. Nährmedium nach Anspruch 1, wobei die Kohlenstoffpartikel einen durchschnittlichen Durchmesser zwischen etwa 100 nm und etwa 400 nm aufweisen.

6. Nährmedium nach Anspruch 1, wobei das Medium ferner den Farbstoff umfasst, wobei der Farbstoff optional einen Azofarbstoff umfasst, wobei der Azofarbstoff optional Acid Black 71, Acid Black 194, Acid Black 2, Reactive Black 5, Direct Black 36, oder Nyanza Black ist.

7. Nährmedium nach Anspruch 1, das ferner ein Polymer umfasst, das an das mindestens eine Pigment und optional mindestens einen Farbstoff bindet, wobei das Polymer optional Polydiallyldimethylammoniumchlorid (PolyDADMAC), Stärke, Cellulose, Chitosan, β-Cyclodextrin, Polymere auf Calix[4]aren-Basis, oder ein Polymer auf p-*tert*-Butylcalix[4]aren-Basis umfasst.

8. Nährmedium nach Anspruch 1, das ferner einen Radikalfänger umfasst, wobei der Radikalfänger optional aus der Gruppe ausgewählt ist, die aus N-Acetyl-L-cystein, Natriumascorbat, Thioharnstoff, Nicotinamid, Pyridoxin, Natriumpyruvat, D-Mannitol, 2-Oxo-4-thiomethylbuttersäure (OBTA), 3-(Methylthio)propionaldehyd (Methional), Dimethylsulfoxid (DMSO), tert-Butylalkohol, Benzoat, Katalase, NADH-Peroxidase, Thiolperoxidase, Bakterioferritin-Komigrationsprotein, Alkylhydroperoxid-Reduktase, Glutathionperoxidase, Cytochrom-c-Peroxidase, und Rubrerythrin ausgewählt ist.

9. Nährmedium nach Anspruch 1, wobei der Mikroorganismus ein Bakterium, Pilz, Protist, oder Archaea ist, wobei der Mikroorganismus optional zu einer Gattung gehört, die aus der Gruppe ausgewählt ist, die aus *Acinetobacter, Aspergillus, Bacillus, Burkholderia, Campylobacter, Candida, Clostridium, Corynebacterium, Delftia, Dermacoccus, Escherichia, Exserohilum, Helicobacter, Kocuria, Legionella, Listeria, Methylobacterium, Micrococcus, Mycobacterium, Paenibacillus, Penicillium, Propionibacterium, Pseudomonas, Ralstonia, Salmonella, Selenomonas, Serratia, Shigella, Staphylococcus, Stenotrophomonas, Streptococcus, Streptomyces, Vibrio* und *Yersinia.*

10. Nährmedium nach Anspruch 1, wobei das Geliermittel aus der Gruppe ausgewählt ist, die aus Agar, Gellan, Natriumalginat, Xanthangummi, Guargummi, Gelatine, Agarose, Polyacrylamid, und einem von *Rhizobium sp.* (CNCM-Nummer: I-1809) produzierten Polysaccharid besteht.

11. Nährmedium nach Anspruch 1, das ferner Nährstoffe umfasst, die aus der Gruppe ausgewählt sind, die besteht aus Trypticase-Soja-Medium (TSM), flüssigem Thioglykolat-Medium (FTM), Schaedler-Medium, Lysogeny/Lennox (LB)-Bouillon, Mannitol-Hefeextrakt-Pepton-Bouillon, Terrific Bouillon II, Super Optimal Broth mit Katabolitenrepression (SOC)-Medium, Thornton-Medium, Lowenstein-Jensen-Medium, Sabouraud-Dextrose-Medium, D/E-Neutralisationsmedium, Loeffler-Medium, Nalidixinsäure- und Colimycin (ANC)-Medium, künstliches Meerwassermedium, Bismutsulfit-Agar, Chapman-Agar, Mueller-Hinton-Agar (MHA), Blutagar, MacConkey-Agar, Trypticase-Soja-Agar (TSA), Sabouraud-Dextrose-Agar (SDA), Reasoner's 2A-Agar (R2A), Mueller-Miller-Tellurit-Agar, oder Tinsdale-Tellurit-Agar.

12. Nährmedium nach einem der Ansprüche 1 bis 11, wobei der Nährstoff fest oder halbfest ist.

13. Nährmedium nach einem der Ansprüche 1 bis 11, wobei das Nährmedium flüssig ist, wobei das Nährmedium optional in einer porösen Matrix angeordnet ist.

14. Verfahren zum Nachweis des Wachstums eines Mikroorganismus, umfassend:
(a) Inkontaktbringen einer Oberfläche des Nährmediums nach Anspruch 12 mit einer Probe; und
(b) optisches Nachweisen von Fluoreszenz auf der Oberfläche, um ein Wachstum des Mikroorganismus festzustellen.

15. Verfahren nach Anspruch 14, wobei Schritt (a) das Inkontaktbringen einer die Probe enthaltenden Membran mit der Oberfläche umfasst, oder wobei Schritt (a) das Inkontaktbringen einer Außenfläche mit der Oberfläche umfasst, und/oder wobei Schritt (a) das Inkontaktbringen von Luft mit der Oberfläche umfasst.

## Revendications

1. Milieu nutritif liquide, solide ou semi-solide comprenant au moins un pigment et éventuellement au moins un colorant,
dans lequel le milieu nutritif favorise la croissance d'un micro-organisme, dans lequel le au moins un pigment et éventuellement au moins un colorant réduisent sensiblement le fond du milieu, et dans lequel le milieu nutritif solide ou semi-solide comprend en outre un agent gélifiant, dans lequel le milieu comprend le pigment, dans lequel le pigment comprend des particules de carbone ou des particules d'oxyde de fer, dans lequel les particules de carbone ont un diamètre moyen inférieur à 500 nm.

2. Milieu nutritif selon la revendication 1, dans lequel le milieu nutritif est disposé dans un récipient, dans lequel, éventuellement, le milieu nutritif est proéminent par rapport au récipient, dans lequel, éventuellement, le milieu nutritif est arrondi.

3. Milieu nutritif selon la revendication 1, dans lequel le au moins un pigment et éventuellement au moins un colorant, réduit sensiblement le fond rouge, le fond orange, le fond jaune, le fond vert, le fond bleu ou le fond violet résultant d'une exposition à la lumière blanche, ou le fond résultant d'une exposition à la lumière bleue.

4. Milieu nutritif selon la revendication 1, dans lequel le au moins un pigment et éventuellement au moins un colorant fournissent une densité optique maximale comprise entre 0,004 et 6.

5. Milieu nutritif selon la revendication 1, dans lequel les particules de carbone ont un diamètre moyen compris entre environ 100 nm et environ 400 nm.

6. Milieu nutritif selon la revendication 1, dans lequel le milieu comprend en outre le colorant, dans lequel, éventuellement, le colorant comprend un colorant azoïque, dans lequel, éventuellement, le colorant azoïque est le noir acide 71, le noir acide 194, le noir acide 2, le noir réactif 5, le noir direct 36 ou le noir Nyanza.

7. Milieu nutritif selon la revendication 1, comprenant en outre un polymère qui se lie à au moins un pigment et éventuellement à au moins un colorant, dans lequel, éventuellement, le polymère comprend du chlorure de polydiallyldiméthylammonium (polyDADMAC), de l'amidon, de la cellulose, du chitosane, de la β-cyclodextrine, des polymères à base de calix[4]arène ou un polymère à base de p-tert-butylcalix[4]arène.

8. Milieu nutritif selon la revendication 1, comprenant en outre un piégeur de radicaux libres, dans lequel, éventuellement, le piégeur de radicaux libres est choisi dans le groupe constitué par la N-acétyl-L-cystéine, l'ascorbate de sodium, la thiourée, la nicotinamide, la pyridoxine, le pyruvate de sodium, le D-mannitol, l'acide 2-oxo-4-thiométhylbutyrique (OBTA), 3-(méthylthio)propionaldéhyde (méthional), diméthylsulfoxyde (DMSO), alcool tert-butylique, benzoate, catalase, NADH peroxydase, thiol peroxydase, protéine comigratoire bactérioferritine, alkyl hydroperoxyde réductase, glutathion peroxydase, cytochrome c peroxydase et rubrerythrine.

9. Milieu nutritif selon la revendication 1, dans lequel le micro-organisme est une bactérie, un champignon, un protiste ou une archée, dans lequel, éventuellement, le micro-organisme appartient à un genre choisi dans le groupe constitué par Acinetobacter, Aspergillus, Bacillus, Burkholderia, Campylobacter, Candida, Clostridium, Corynebacterium, Delftia, Dermacoccus, Escherichia, Exserohilum, Helicobacter, Kocuria, Legionella, Listeria, Methylobacterium, Micrococcus, Mycobacterium, Paenibacillus, Penicillium, Propionibacterium, Pseudomonas, Ralstonia, Salmonella, Selenomonas, Serratia, Shigella, Staphylococcus, Stenotrophomonas, Streptococcus, Streptomyces, Vibrio et Yersinia.

10. Milieu nutritif selon la revendication 1, dans lequel l'agent gélifiant est choisi dans le groupe constitué par l'agar, le gellan, l'alginate de sodium, la gomme xanthane, la gomme guar, la gélatine, l'agarose, le polyacrylamide et un polysaccharide produit par Rhizobium sp. (numéro CNCM : I-1809).

11. Milieu nutritif selon la revendication 1, comprenant en outre des nutriments choisis dans le groupe constitué par le milieu trypticase soja (TSM), le milieu thioglycolate fluide (FTM), le milieu de Schaedler, bouillon de lysogénie/Lennox (LB), bouillon de peptone d'extrait de levure de mannitol, bouillon terrific II, bouillon Super Optimal avec répression catabolique (SOC), milieu de Thornton, milieu de Lowenstein-Jensen, milieu de Sabouraud dextrose, milieu neutralisant D/E, milieu Loeffler, milieu à l'acide nalidixique et à la colimycine (ANC), milieu à eau de mer artificielle, gélose au sulfite de bismuth, gélose Chapman, gélose Mueller-Hinton (M HA), gélose au sang, gélose MacConkey, gélose trypticase soja (TSA), gélose Sabouraud dextrose (SDA), gélose Reasoner 2A (R2A), gélose Mueller-Miller au tellurite ou gélose Tinsdale au tellurite.

12. Milieu nutritif selon l'une quelconque des revendications 1 à 11, dans lequel le nutriment est solide ou semi-solide.

13. Milieu nutritif selon l'une quelconque des revendications 1 à 11, dans lequel le milieu nutritif est liquide, dans lequel, éventuellement, le milieu nutritif est disposé à l'intérieur d'une matrice poreuse.

14. Procédé de détection de la croissance d'un micro-organisme comprenant :
(a) la mise en contact d'un échantillon avec une surface du milieu nutritif selon la revendication 12 ; et
(b) détecter optiquement la fluorescence à la surface afin de déterminer toute croissance du micro-organisme.

15. Procédé selon la revendication 14, dans lequel l'étape (a) comprend la mise en contact d'une membrane contenant l'échantillon avec la surface, ou dans lequel l'étape (a) comprend la mise en contact d'une surface externe avec la surface, et/ou dans lequel l'étape (a) comprend la mise en contact de l'air avec la surface.
